# EUROPEAN PATENT APPLICATION

(11) **EP 0 596 700 A1**
(43) Date of publication of application: **11.05.1994**
(21) Application number: 93308746.2
(22) Date of filing: 02.11.1993
(51) Int. Cl.: G01N 33/52, G01N 33/66, C09B 69/10, C09B 46/00, C09B 31/00, A61L 2/00

(54) **Cis-diol detection method and solid supports**

(30) Priority: 03.11.1992 US 970661
(71) Applicant: Hewlett-Packard Company, Palo Alto, California 94304 (US)
(72) Inventor: Robotti, Karla M., Foster City, CA 94304 (US)
(74) Representative: Williams, John Francis

(57) **Abstract**

The present invention is directed to methods and kits for measurement of cis-diols in a sample by means of dyes which produce a spectral change upon reaction or complexation with cis-diols. The present invention is also directed to solid supports having such dyes as substituents thereon. In particular, measurement of glucose using azo boronic dyes is disclosed.

## Description

The present invention is directed to methods and kits for measurement of cis-diols in a sample by means of dyes which produce a spectral change upon reaction or complexation with cis-diols. The present invention is also directed to solid supports having such dyes as substituents thereon.

### References

The following references are cited in the application as superscript numbers at the relevant portion of the application:
1. Mansouri, S.; Schultz, J., *Bio/Technology,* 1984, October, 885-890.
2. Narayanaswamy, R.; Sevilla, F., *Anal.* *Letters,* 1988, 21 (7), 1165-1175.
3. Goldfinch, M.J.; Lowe, C.R., *Anal. Biochem.,* 1984, 138, 430-436.
4. Bradley, J.; Kidd, A.; Anderson, P.A.; et al., *Analyst,* 1989, 114, 375-379.
5. Mascini, M.; Selleri, S., *Anal. Letters,* 1989, 22(6), 1429-1449.
6. Abdulla, H.; Greenway, G.; Platt, A.; Fielden, P.; *Analyst,* 1989, 114, 785-788.
7. Gunasingham, H.; Tan, C.H.; Seow, J., *Anal.* *Chem.*, 1990, 62, 755-759.
8. Moreno-Bondi, M.; Wolfbeis, O.; Leiner, M.; Schaffar, B., *Anal. Chem.,* 1990, 62, 2377-2380.
9. Weith, H.; Wiebers, J.; Gilham, P., *Biochemistry,* 1970, 9, 4396-4401.
10. Rosenberg, M.; Wiebers, J.; Gilham, P., *Biochemistry,* 1972, 11, 3623-3628.
11. Wulff, G.; Vesper, W.; Grobe-Einsler, R.; Sarhan, A., *Makromol. Chem.,* 1977, 178, 2799-2816.
12. Wulff, G.; Sarhan, A.; Zabrocki, K., *Tetrahedron Lett.,* 1973, (44), 4329-4332.
13. Cockbain, J., International Publication No. W092/08722, 1992.
14. Pawlowski, N.; Russell, D.; Robotti, K.; U.S. Patent No. 5,108,502, 1992.
15. Shea, K.; Thompson, E.; Pandey, S.; Beauchamp, P., *J.* Am. *Chem. Soc.,* 1980, 102, 3149-3155.
16. Shea, K.; Thompson, E., *J.* *Org*. *Chem.,* 1978, 43, 4253-4255.
17. Shea, K.; Dougherty, T., *J. Am. Chem.* *Soc.*, 1986, 108, 1091-1093.
18. Shea, K.; Sasaki, D., *J.* *Am. Chem. Soc.*, 1989, 111, 3442-3444.
19. Weetall, U.S. Patent No. 3,652,761.
20. Blakemore, et al., U.S. Patent No. 4,088,746.

The disclosure of the above patents and publications are herein incorporated by reference in their entirety to the same extent as if the language of each individual publication, patent and patent application were specifically and individually included herein.

### State of the Art

The problem of detecting cis-diols, in particular glucose, in fermentation systems or bioreactors in the pharmaceutical, food or biotechnology industries can be critical. Presently, there is no real-time, on-line method for the determination of cis-diols in fermenters. In order to be most useful in a fermentation system, a detection method must be able to undergo steam sterilization.

There are known glucose sensors which involve the use of enzymes, usually glucose oxidase, as part of the determination method¹⁻⁸. Since such systems are incapable of withstanding steam sterilization, their usefulness for measuring glucose in a fermentation system is limited.

It is also known that glucose may be detected by measuring optical rotation. However, this physical method requires low signal response from other optically-active compounds and low scattering media. These conditions are unlikely in a typical fermenter.

Therefore, it is apparent that there is a need for a method and means for quantitively measuring cis-diols in various environments, including fermentation processes.

### Summary of the Invention

The present invention is directed to the recognition that certain dyes, upon reaction or complexation with cis-diols, change spectrally, and that this spectral change can be measured to determine the amount of cis-diols present in a sample. These dyes may also be bound permanently to a solid support without interfering with their cis-diol sensitivity. No enzymes or protein-based components are required. Thus, the present invention provides an optic-based, non-enzymatic, steam-sterilizable detection method for cis-diols.

Accordingly, in one of its aspects, the present invention is directed to a method of measuring cisdiols in a sample, using dyes which give a spectral change when reacted or complexed with cis-diols.

The present invention is also directed to a solid support having dye substituents thereon. Such solid supports are represented by the following formula: SU - [ R₂ - ( - N = N - R₁ - )ₘ - N = N - R - (B(OH)₂)ₙ ]ₓ where SU is a solid support; R is a phenylene or substituted phenylene; R₁ is an unsubstituted arylene or a substituted arylene; R₂ is an unsubstituted aryl or a substituted aryl; m is 0 or an integer from 1 to about 4, n is an integer from 1 to 2, and x is an integer equal to at least 1.

In a further composition aspect, the present invention is directed to kits for measuring cis-diols in a sample. Such kits include a suitable dye, means for contacting the dye with the sample to be measured, and means for measuring the spectral change of the dye.

### BRIEF DESCRIPTION OF THE FIGURES

The accompanying figures, which are incorporated in and form a part of this specification, illustrate embodiments of the invention and, together with the description, serve to explain the principles of the invention.

Figures 1A and 1B illustrate a series of dyes useful in the present invention.

Figure 2 illustrates absorbance difference spectra using Compound 7 in solution.

Figure 3 illustrates a calibration curve for Compound 7.

Figure 4 illustrates absorbance spectra using Compound 20 in solution.

Figure 5 illustrates a calibration curve for Compound 20.

Figure 6 illustrates absorbance spectra using Compound 21 in solution.

Figure 7 illustrates absorbance difference spectra using Compound 26 in a fiber optic probe.

### DETAILED DESCRIPTION OF THE INVENTION

As noted above, the present invention is directed in part to methods and kits using dyes to measure cis-diols in a sample. The present invention is also directed to solid supports containing such dyes.

### A. Definitions

Prior to discussing this invention in further detail, the following terms will first be defined.

As used herein, the terms "cis-diol" or "cisdiols" refer to dihydroxy alcohol(s) containing two hydroxyl groups connected to adjacent carbon atoms where both hydroxyl groups are on the same side of the molecule. This includes glycols, such as ethylene glycol, and some sugars, such as glucose.

The terms "support" and "solid support" refer to any support which contains surface reactive groups or can be derivatized so as to contain surface reactive groups which can react with the functional group on the dye so as to form a stable covalent bond. Suitable solid supports may be non-polymeric as well as polymeric. They include sepharose (including Sepharose 4B), cellulose, aminoethyl cellulose, glass, silica, aminopropyl-silica, aminopropyl-CPG (controlled pore glass), glass beads, Trisacryl-NH, polyacrylamide particles, various membranes, and the like. Such supports are either commercially available or can be prepared using conventional methodology.

The terms "azo boronic dye", "azo boronic acid dye", and "boronic acid dye" refer to azo dyes which contain at least one boronic acid moiety on an aryl ring.

The term "spectrophotometer" refers to any device that measures absorption or emission at one or more wavelengths, and includes diodes which measure absorption at only a small portion of the spectrum.

### B. Measurement of cis-Diols

The present invention provides a unique photometric method of detecting cis-diols. Many sugars, including glucose, are cis-diols. This method uses the spectral change of a dye when the dye reacts with a cis-diol as a measure of the amount of cis-diol present. In many fermentations of a pharmaceutical or biotechnological nature, the only cis-diol present in any appreciable extent is glucose.

The useful detection range for a glucose determination method in such situations is expected to be from about 1 to 100 mg/ml, with resolution of about 0.5 mg/ml preferred.

A useful cis-diol determination method must be able to function in a range of pHs. A useful working pH range is from about 3 to 10. The method of the present invention is relatively unaffected by pH changes, and can measure cis-diols in a pH range from about 3 to 10.

The ability to undergo steam sterilization (120°C for 20 min.) is a requirement for any in situ fermentation probe. Since the method of the present invention is not enzymatic or protein-based and these dyes are stable under such conditions, this requirement will be met.

Any dye which is capable of reacting or complexing with cis-diols such that a spectral change of the dye occurs is useful in the present invention. It is known in the art that aryl boronic acid conjugates bind to cis-diols, including glucose⁹⁻¹². There is one reported case in which the boronic acid conjugate was contained within a dye structure¹³. In this case, the dye was used as a labelling agent; the detection of cis-diols was not achieved by spectral change determination. Arylboronic acids have been used on affinity chromatography columns and other applications for binding cis-diols. In particular, certain azo boronic dyes have been found to be useful in the present invention, since they react or complex with cis-diols and show a spectral change which is at an unusual part of the spectrum. Thus, other components of the fermentation media which might be present would not interfere with the spectrophotometric determination of cis-diols, since they would not have absorption maxima at these wavelengths. Additionally, uncomplexed dye would not interfere.

While not meant to limit the invention in any way, the mechanism of action of some of the dyes useful in the present invention is described below to elucidate in detail how the method of the present invention may work.

An arylboronic acid (1), hydrates to structure 2 in aqueous solution. The addition of a cis-diol, such as glucose, produces the complex (3) with the loss of two molecules of water. The aryl portion (Ar) of 1 which is usually a simple phenyl ring, was replaced with an extended conjugated system such as 4. It has been found that complexation of a cis-diol with an azo boronic dye containing such an extended conjugation system resulted in a change of the visible spectra of compound 1.

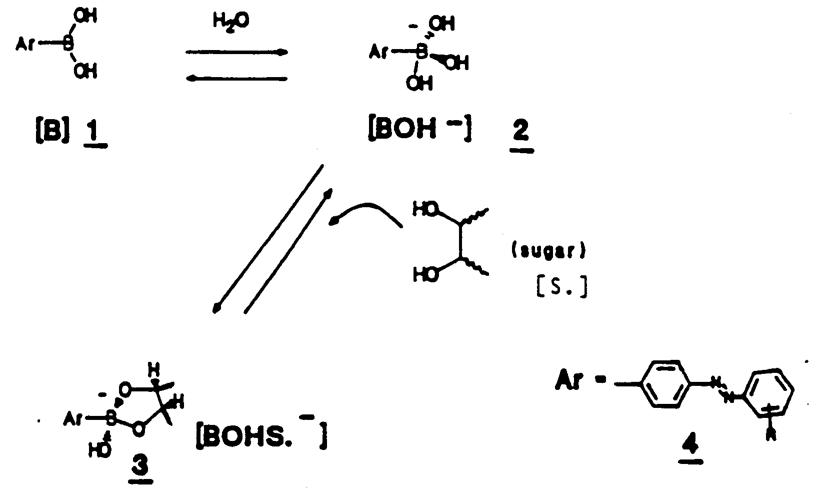

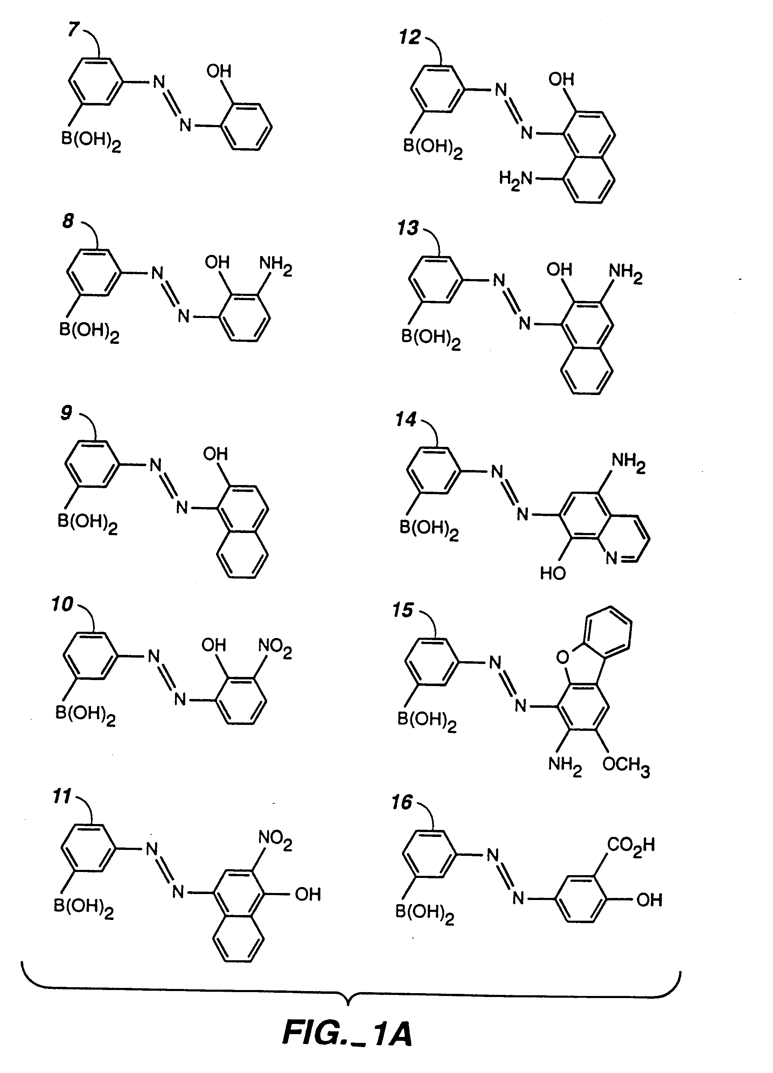

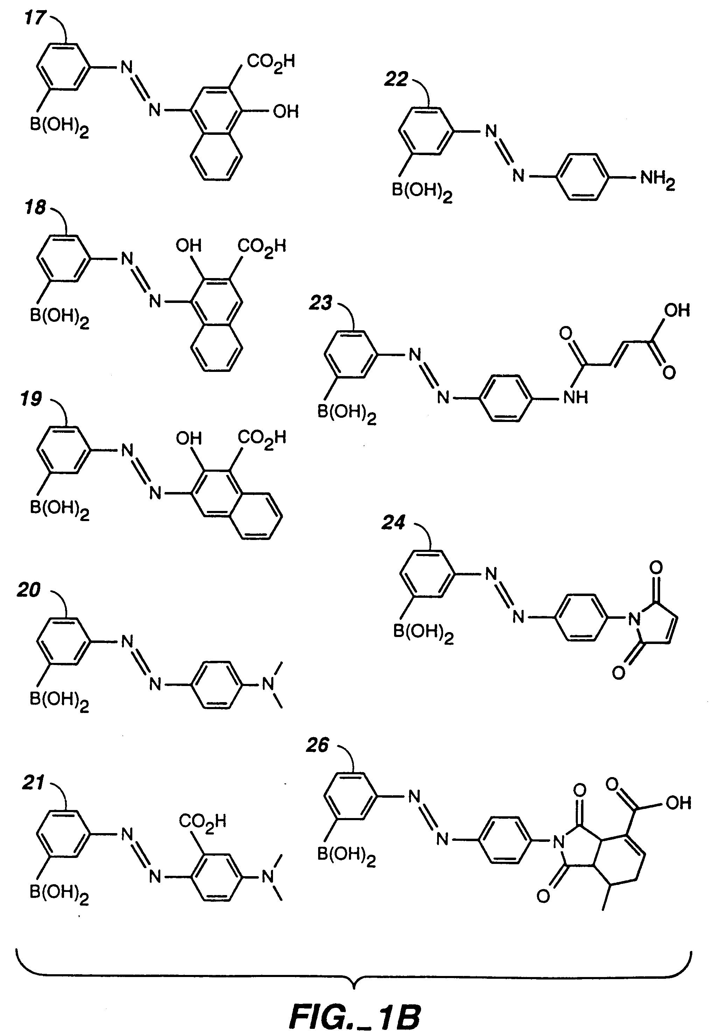

A series of azo boronic acid dyes were prepared, as shown in Figures 1A and 1B. The synthesis of these azo dyes is described in U.S. Patent No. 5,108,502, issued to Pawlowski, et al., which is incorporated herein by reference¹⁴. Other dyes which may be useful in the present invention because they undergo a spectral change when they react or complex with cis-diols are either commercially available or can be prepared using conventional methodology.

It is expected that polymer templates for glucose (or other specific cis-diols) will also be useful in the methods of the present invention. The preparation and use of such polymer templates to orient molecules in a specific stereochemistry or introduce clusters of functional groups is known in the art^{11. 12. 15-18}. For example, by complexing two vinyl phenylboronic acid monomers with glucose and copolymerizing the complex with an excess of cross-linking agent, a polymer backbone will be formed. Hydrolyzing the glucose away from the polymer with an acid wash will leave a polymer template with binding sites that are specific for glucose. Complexation of the polymer with glucose will create a spectral change. This sort of polymer template may be created for detecting a particular cisdiol of interest, such as glucose, rather than all cis-diols.

Dyes useful in the method of the present invention may be coupled to a solid support. The solid support may be appropriately functionalized. This coupling reaction is conducted by employing complimentary reactive functionalities on the dye and on the solid support. That is to say that the surface reactive group employed on the solid support is one which is reactive with the functional group on the dye so as to form a stable covalent bond. For example, if an amine group is employed as the surface reactive group on the solid support, then the functional group on the dye should be a group which is reactive with an amine (e.g., a carboxylic acid or its activated ester or mixed anhydride). Such coupling reactions are well known in the art and are described in, for example, Weetall, U.S. Patent No. 3,652,761 (glass beads)¹⁹, Blakemore et al., U.S. Patent No. 4,088,476 (polyacrylamide particles)²⁰, and the like.

The coupling reaction results in the loss or conversion of part of the functional group on the dye. For example, if the functional group is a carboxyl group which is reacted with an amine, then the resulting amide will have only the residual carbonyl group from the carboxyl functional group on the dye. In these circumstances, the -OH group of the functional group attached to the dye is lost during coupling, resulting in only a residual carbonyl group.

The coupling reaction is generally conducted by adding a molar equivalent amount or a substantial molar excess of the functionalized dye of this invention to a composition containing the solid carrier under conditions whereby the functional group(s) on the dye react with a surface reactive group on the solid support. The amount of functionalized dye added in conjunction with the number of reactive sites on the solid support dictates the number of functionalized dye substituents attached to each solid support. In general, sufficient functionalized dye is added so as to provide a measurable spectral change when reacted or complexed with cis-diols.

The resulting solid support having one or more dye substituents bound thereto through a stable covalent bond are particularly useful for providing in situ cis-diol measurements which can have a variety of uses. The solid support having one or more dye substituents bound thereto can be used as a non-bleeding glucose sensor incorporated into solid phase detection devices such as dipsticks, fiber optic probes or sensors. Alternatively, if the solid supports have particle sizes which form a substantially stable suspension in an aqueous solution, the cis-diol concentration of an incubation, a fermenter, etc. can be continuously monitored on-line. Further, after incubation, fermentation, etc., the particles can be readily removed by centrifugation. Other uses for these solid phase indicators will be readily apparent to those skilled in the art.

A preferred embodiment of a solid support according to the present invention is a fiber optic probe wherein a certain amount of the dye which is immobilized on a solid support is fixed at the common end of a bifurcated fiber optic bundle in such a way that it can be exposed to liquid samples. The support can be contained in a mesh, or in a membrane that can help isolate the support from potentially interfering compounds. Light that is directed to the solid support can either be scattered back into the bundle by the support material, or can be reflected back by a polished surface after passing through the material. This returned light is measured by a spectrometer or filter photometer to obtain the desired signal. Instead of a bundle, a single optical fiber can be used to transfer light both to and from the support material if used with the appropriate beamsplitting devices.

Another preferred embodiment of a solid support according to the present invention is a membrane having one or more dye substituents bound thereto.

The dyes useful in the present invention may be included as part of a kit used to measure cis-diols in a sample. Such kits also include means for contacting the dye with the sample to be measured and means for measuring the spectral changes of the dye.

Means for contacting the dye with the sample may include withdrawing an aliquot of the solution to be measured and mixing it with the dye, also in solution. Alternatively, the aliquot may be placed in contact with the dye which is on a solid support. Other possible means for contacting the dye with the sample include placing a probe containing the dye on a solid support in the solution to be measured. The latter means allows for continuous real-time measurement of cis-diols. Real-time measurement of cis-diols may also be accomplished using flow injection analysis techniques using bound or unbound dye.

Means for measuring the spectral change of the dye may include comparing the color of the dye and sample solution to a color chart, an optical spectrometer, a spectrophotometer, a linear variable filter, a filter photometer, and fiber optic cabling leading to a spectrophotometer.

The following examples are offered to illustrate this invention and are not to be construed in any way as limiting the scope of this invention.

### EXAMPLES

Azo boronic dyes were prepared following the methods of Pawlowski, et al. (U.S. Patent No. 5,108,502). Routinely, reaction courses and product mixtures were monitored by thin layer chromatography using Baker-flex silica gel IB-F sheets. Plates were visualized by iodine vapor or UV light. Ultraviolet visible (UV-VIS) spectra were recorded with a HP 8452 diode array spectrophotometer. All chemicals were purchased from Aldrich Chemical Company or Sigma and used without further purification. Sepharose gels were purchased from Pharmacia while aminoethyl cellulose was obtained from Serva Biochemicals. Evaporation under vacuum was carried out using Speed Vac Systems (Savant Instruments, Inc., Farmingdale, N.Y.).

### Example 1 - Measurement of glucose using 3-((2-hydroxyphenyl)azo) benzene boronic acid (Compound 7 in Figure 1A)

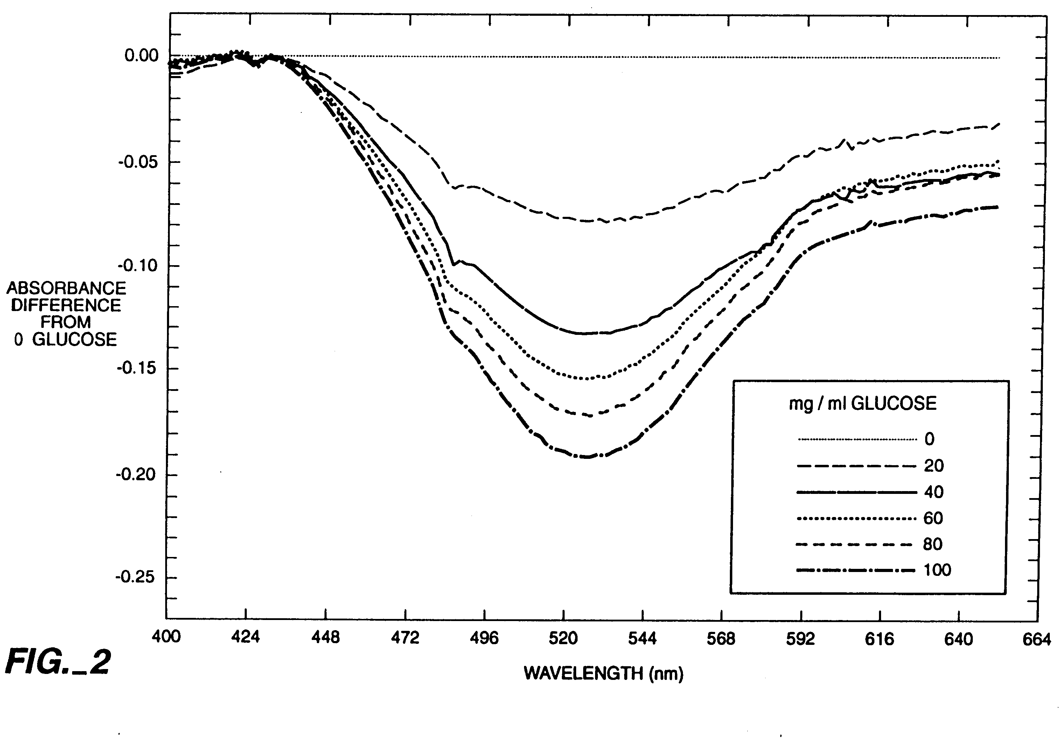

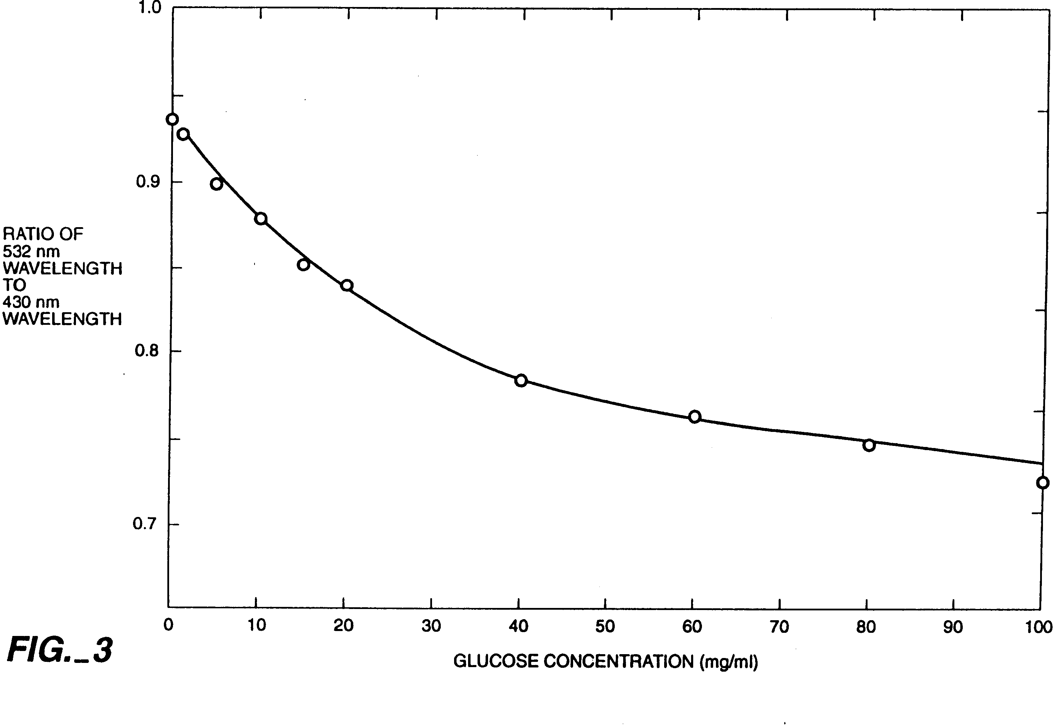

3-((2-hydroxyphenyl)azo) benzene boronic acid (Compound 7 in Figure 1A) was prepared by the method of Pawlowski et al. A stock solution of 6 mg/ml in ethanol was prepared. Stock solutions of 0, 20, 40, 60, 80 and 100 mg/ml glucose in pH 7 phosphate buffer were prepared. To 3 ml of each glucose stock solution, 50 µl dye stock solution was added. The absorbance spectra of the resulting solutions was measured. Figure 2 shows the resulting absorbance spectra of the stock glucose solutions minus the absorbance in 0.0 mg/ml glucose. Figure 3 shows the glucose calibration curve for Compound 7.

### Example 2 - Measurement of glucose using 3-(((4-N,N-dimethyl)aminophenyl)azo) benzene boronic acid (Compound 20 in Figure 1B)

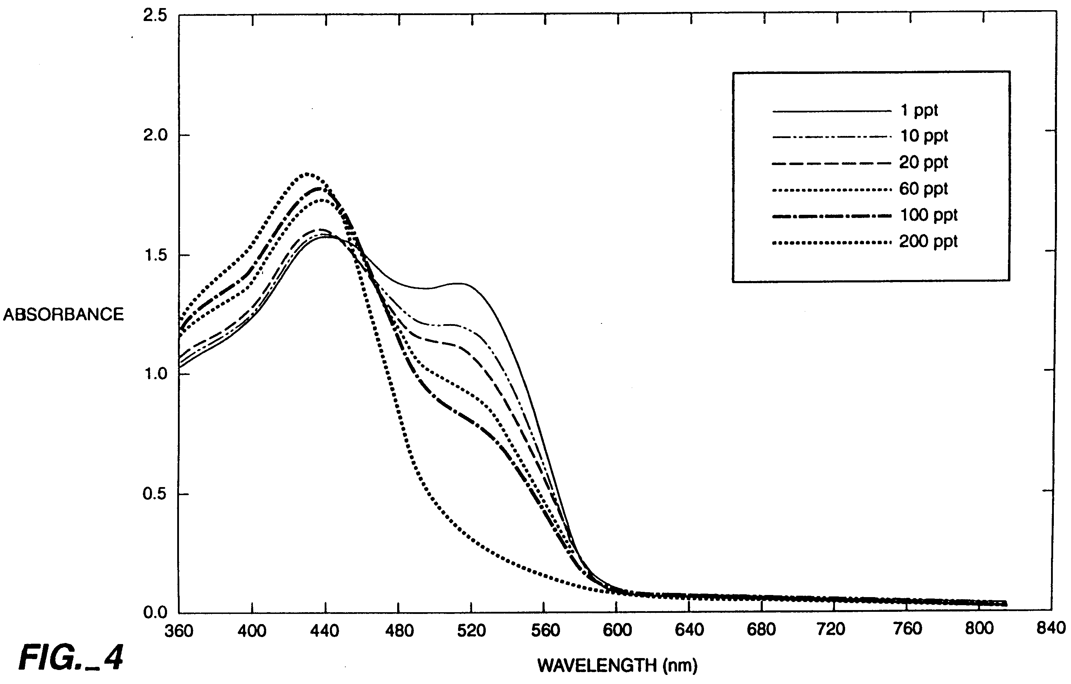

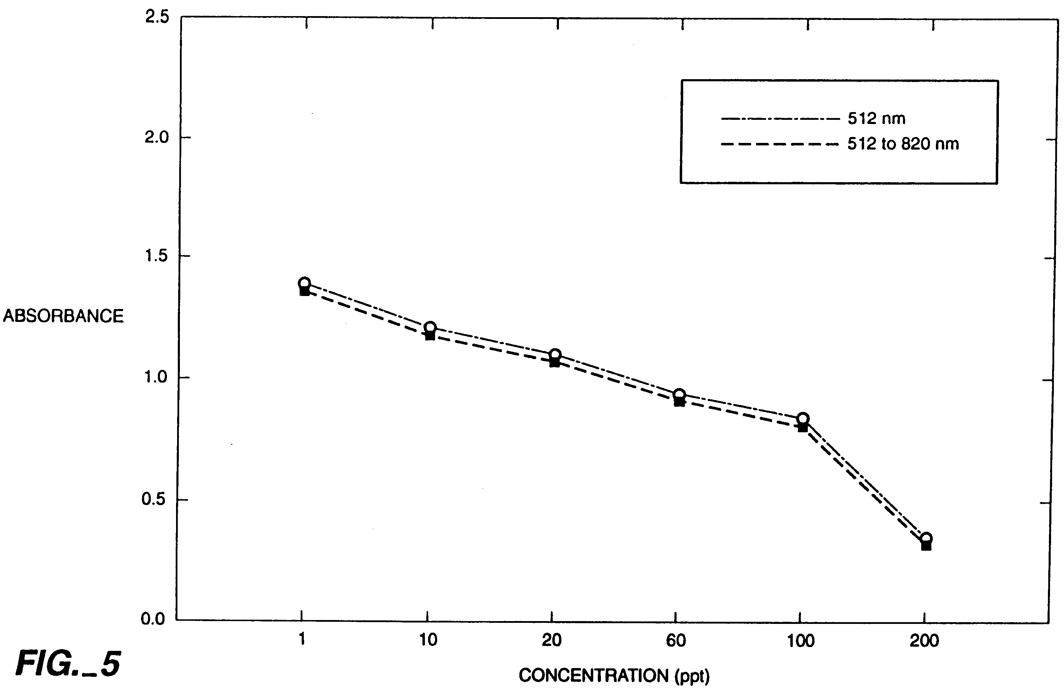

Compound 20 was prepared by the method of Pawlowski, et al. A stock solution of 6 mg/ml in ethanol was prepared, and 50 µl added to 3 ml of the following glucose stock solutions in pH 7 phosphate buffer: 20, 60, 100 and 200 parts per thousand (ppt). The absorbance spectra of the resulting solutions was measured. Figure 4 shows the resulting absorbance spectra and Figure 5 shows the resulting glucose calibration curve for Compound 20.

### Example 3 - Measurement of glucose using 3-(((4-N,N-dimethyl)amino-2-carboxyphenyl)azo) benzene boronic acid (Compound 21 of Figure 1B)

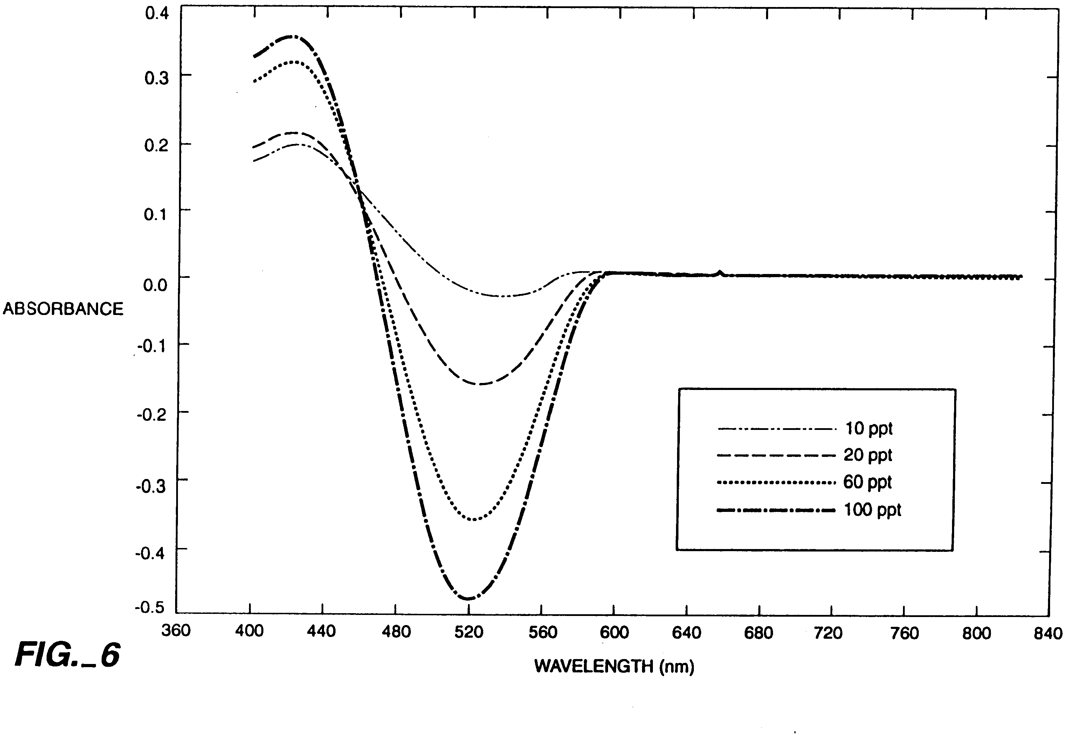

Compound 21 was tested in solution against glucose standards following the procedures described in Examples 1 and 2 above. Figure 6 shows the resulting absorbance spectra of the glucose solutions.

Compound 21 was coupled to aminoethyl cellulose using the following procedure. Aminoethyl cellulose (0.5g) was swelled in 30 ml DI water for 1 hour, collected and put aside. In a separate beaker, 1.3 mmol of Compound 21 was dissolved in 1 ml DMF and cooled to 0°C in an ice-ethanol bath. Isobutyl chloroformate (0.23 ml, 1.8 mmol) and N-methylmorpholine (0.2 ml, 1.8 mmol) were added and the reaction was left at 0°C for 15-20 min.

The aminoethyl cellulose gel was added to the reaction along with about 10 mg of benzyltributyl ammonium bromide and 30 ml borate buffer (pH 9). The reaction flask was placed on a shaker overnight at room temperature. The support was collected on a frit and washed with volumes of water, ethanol, acetone and buffered water.

The support was placed in a fiber optic probe and tested against stock glucose solutions. Absorption difference spectra matched the spectra in free solution.

### Example 4 - Measurement of glucose using 3-((4-1,4,5,6-tetrahydro-2-carboxyl-5-methylphthalimido) phenyl)azo)benzene boronic acid (Compound 26 of Figure 1B)

Compound 26 was tested in solution against glucose standards following the procedures described in Examples 1 and 2. Unbound, it showed less response than Compounds 7, 20 or 21.

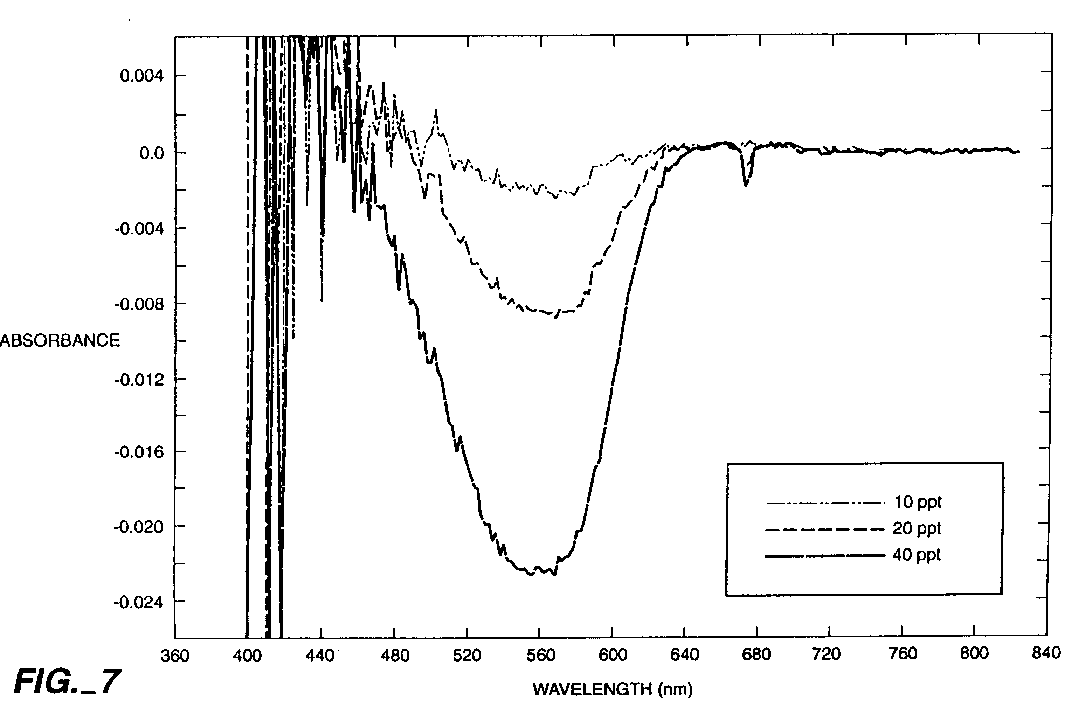

Compound 26 was then coupled to aminoethyl cellulose following the procedures described in Example 3 and tested in a fiber optic probe. Figure 7 shows the absorbance difference spectra of stock glucose solutions using a fiber optic probe containing Compound 26 bound to aminoethyl cellulose.

Additional tests of Compound 26 at a pH range of 4 through 9 showed that it was not sensitive to pH changes.

It will be apparent to one of ordinary skill in this art that various changes and modification of an obvious nature may be made without departing from the spirit of the invention, and all such modifications are considered to fall within the scope of the invention, as defined by the appended claims.

## Claims

1. A method of measuring cis-diols in a sample, comprising the steps of:
a) contacting said sample with a dye capable of reacting or complexing with a cis-diol to thereby produce a spectral change of said dye; and
b) measuring said spectral change of said dye to thereby determine an amount of cis-diols in said sample.

2. A method according to claim 1 further characterized in that said dye comprises a compound according to the following formula:
R₂ - ( - N = N - R₁ - )ₘ - N = N - R - ( B(OH)₂)ₙ
wherein R is a phenylene or substituted phenylene; R₁ is an unsubstituted arylene or a substituted arylene; R₂ is an unsubstituted aryl or a substituted aryl; m is 0 or an integer from 1 to about 4, and n is an integer from 1 to 2.

3. A method according to claim 1 further characterized in that at least a portion of said dye is in solution.

4. A method according to claim 1 further characterized in that said cis-diol to be measured comprises a specific cis-diol and said dye comprises a polymer template for said specific cis-diol.

5. A solid support having one or more dye substituents thereon according to the following formula:
SU-[ R₂ - (- N = N - R₁-)ₘ - N = N - R - (B(OH)₂)ₙ ]ₓ
wherein SU is a solid support; R is a phenylene or substituted phenylene; R₁ is an unsubstituted arylene or a substituted arylene; R₂ is an unsubstituted aryl or a substituted aryl; m is 0 or an integer from 1 to about 4, n is an integer from 1 to 2, and x is a positive integer.

6. A solid support according to claim 5 wherein SU comprises Sepharose 4B, aminoethyl cellulose, glass, silica, or a membrane.

7. A kit for measuring cis-diols in a sample, comprising:
a) a dye capable of reacting or complexing with a cis-diol to thereby produce a spectral change of said dye;
b) means for contacting said dye with said sample; and
c) means for measuring said spectral change of said dye to determine an amount of cis-diols in said sample.

8. An invention according to any of claims 1 to 7 wherein said cis-diol comprises glucose.

9. An invention according to any of claims 1 to 7 wherein said dye comprises an azo boronic dye.

10. An invention according to any of claims 1 to 7 wherein at least a portion of said dye is attached to a solid support.

11. An invention according to any of claims 1, 5 or 7 wherein said dye is sterilizable.
